# EUROPEAN PATENT APPLICATION

(11) **EP 1 156 332 A2**
(43) Date of publication of application: **21.11.2001**
(21) Application number: 01303854.2
(22) Date of filing: 27.04.2001
(51) Int. Cl.: G01N 33/573, G01N 33/68, G01N 33/543, G01N 33/50, A61P 25/28

(54) **Assay methods for cyclin dependent kinases**

(30) Priority: 28.04.2000 US 205932 P
(71) Applicant: Pfizer Products Inc., Groton, Connecticut 06340 (US)
(72) Inventor: Foster, Barbara Ann, Pfizer Global Res. and Dev., Groton, Connecticut 06340 (US); Rastinejad, Farzan, Pfizer Global Res. and Dev., Groton, Connecticut 06340 (US)
(74) Representative: Hayles, James Richard

(57) **Abstract**

Disclosed herein are novel methods for measuring the activity of one or more cyclin dependent kinases (CDKs) in a sample, based upon the quantitation of CDK-phosphorylated Rb protein. The method for measuring cyclin-dependent kinase (CDK) activity includes the following: i) contacting the sample with an anti-retinoblastoma protein (Rb) capture antibody that specifically recognizes a CDK-phosphorylated Rb and isolating the capture antibody-Rb complex; ii) contacting the capture antibody-Rb complex with an anti-Rb primary antibody and isolating the capture antibody-Rb-primary antibody complex; and iii) measuring the amount of CDK-phosphorylated Rb in the sample by quantitating the primary antibody present in the capture antibody-Rb-primary antibody complex. These methods may be used to assess intracellular CDK activity in cultured cells or in cells taken from an animal. Also disclosed are methods of identifying an agent that modulates the CDK activity.

SEQUENCE LISTING (Part of the Description)

<110> Pfizer Products Inc.

<120> Assay Methods for Cyclin Dependent Kinases

<130> PC10583

<140> 00000000
<141> 2000-04-28

<160> 4

<170> PatentIn Ver. 2.0

<210> 1
<211> 13
<212> PRT
<213> synthetic construct

<400> 1
Asn Gly Ser Pro Arg Thr Pro Arg Arg Gly Gln Asn Cys
  1               5                  10

<210> 2
<211> 14
<212> PRT
<213> synthetic construct

<400> 2
Phe Glu Thr Gln Arg Thr Pro Arg Lys Ser Asn Leu Asp Cys
  1               5                  10

<210> 3
<211> 14
<212> PRT
<213> synthetic construct

<400> 3
Tyr Leu Ser Pro Val Arg Ser Pro Lys Lys Lys Gly Ser Thr
  1               5                  10

<210> 4
<211> 14
<212> PRT
<213> synthetic construct

<400> 4
Ser Glu Gly Leu Pro Thr Pro Thr Lys Met Thr Pro Arg Ser
  1               5                  10

## Description

### Background of the Invention

This invention relates to methods for measuring the activities of cyclin dependent kinases (CDKs). These kinases regulate the cell cycle and, therefore, play a role in cell growth and division. The methods of the present invention are particularly useful for measuring intracellular CDK activities.

The cell cycle has four defined sequential phases: G1 is the first gap phase in which the cell prepares for DNA replication; S phase is the phase of DNA synthesis during which a complete copy of the entire genome is generated; G2 is the second gap phase in which the cell prepares for division; and lastly, M phase (mitosis) is the period of cell division in which the two copies of DNA segregate to two daughter cells.

In order to regulate cell cycle progression, a CDK must be catalytically activated by forming a complex with a protein known as a cyclin. The expression profiles of different cyclins vary during the course of the cell cycle. Therefore, different CDK/cyclin complexes form and play significant regulatory roles at different stages of the cell cycle (Pavletich, J. Mol. Biol. 287: 821-828, 1999; Morgan, Ann. Rev. Cell Dev. Biol. 13: 261-291, 1997). For example, CDK2, CDK4, and CDK6 each combine with D type cyclins to regulate progression past the GI restriction point, CDK2/cyclin E complexes regulate transition from G1 into S phase, CDK2/cyclin A complexes mediate the transition from S phase to G2, and CDK1 (also known as Cdc2) complexed with cyclin B or cyclin A drives the transition from G2 phase to M phase, and initiates mitosis.

In general, activated CDKs mediate their regulatory functions by phosphorylating cellular proteins, usually on serine or threonine residues. For example, CDK1 (cdc2), CDK2, CDK4, CDK5, and CDK6 phosphorylate the nuclear retinoblastoma (Rb) protein, which leads to transcriptional activation in the cell and the eventual progression into S phase (Knudsen and Wang, J. Biol. Chem. 271: 8313-8320, 1996; Zarkowska and Mittnoch, J. Biol. Chem. 272: 12738-46, 1997; Kitagawa et al., EMBO J. 15: 7060-69, 1996; Connell-Crowley et al., Mol. Cell Bio. 8: 287-301, 1997). Prior to this CDK-mediated phosphorylation, Rb remains in a state of basal phosphorylation. In this basal state, Rb associates with the transcription factor E2F and thereby represses E2F-mediated transcription. However, prior to the onset of DNA synthesis in late G1, activated CDKs hyperphosphorylate Rb. Once hyperphosphorylated, Rb releases E2F, which allows E2F to activate the transcription of cell cycle-regulated genes and commit the cell to enter S phase (Zarkowska and Mittnoch, *supra*; Kitagawa et al., *supra*; Connell-Crowley et al., *supra;* and Lundberg and Weinberg, Mol. Cell Bio. 18: 753-61, 1998).

Perturbations in CDK regulation can result in abnormal cell growth; inhibitors of cellular CDK activity can cause the arrest of the cell cycle (Dynlacht, Nature 389: 149-52, 1997; Fisher, Curr. Opin. Genet. Dev. 7: 32-38, 1997; Morgan, Nature 374: 131-34, 1995; Serrano et al., Nature 366: 704-07, 1993; Kamb et al., Science 264: 436-40, 1994; Nobori et al., Nature 368: 753-56, 1994; Levine, Cell 88: 323-31, 1997; Porter et al., Nature Med. 3: 222-25, 1997; Hunter and Pines, Cell 79: 573-82, 1994; Sherr, Science 274: 1672-77, 1996; Wolfel et al., Science 269: 1281-84, 1995; Zuo et al., Nature Genet. 12: 97-99, 1996; and Sellers et al., Proc. Nat. Acad. Sci. USA 92: 11544-48, 1995). Therefore, compounds that modulate CDK activity are useful in regulating cellular proliferation and may also be useful as therapeutic agents.

The methods currently available for measuring CDK activities and identifying agents that modulate such activities have typically involved measuring CDK phosphorylation of a substrate *in vitro* in the presence and absence of test compounds. However, such tests provide no information regarding whether the test agent has the ability to inhibit any physiologically relevant CDK activity within a cell. Therefore, there is a need for methods to assess the intracellular CDK activities and the ability of agents to modulate such activities.

### Summary of the Invention

The present invention provides novel methods for measuring the activity of one or more cyclin dependent kinases (CDKs) in a sample, based upon the quantitation of CDK-phosphorylated Rb protein. The method for measuring cyclin-dependent kinase (CDK) activity includes the following: i) contacting the sample with an anti-retinoblastoma protein (Rb) capture antibody that specifically recognizes a CDK-phosphorylated Rb and isolating the capture antibody-Rb complex; ii) contacting the capture antibody-Rb complex with an anti-Rb primary antibody and isolating the capture antibody-Rb-primary antibody complex; and iii) measuring the amount of CDK-phosphorylated Rb in the sample by quantitating the primary antibody present in the capture antibody-Rb-primary antibody complex.

In preferred embodiments, the capture antibody is bound to a test plate, preferably a multi-well test plate, the method measures intracellular CDK activity, preferably, the CDK activity of cultured cells or the *ex vivo* activity of a tissue sample taken from an animal. In other preferred embodiments, the method assays CDK2 activity or CDK4 activity, and the CDK activity measured is human.

A second aspect of the present invention features methods of identifying agents that modulate the activity of a CDK in a cell. The method includes i) contacting an agent with the cell; ii) lysing the cell; iii) contacting the cell lysate with an anti-retinoblastoma protein (Rb) capture antibody that specifically recognizes CDK-phosphorylated Rb and isolating the capture antibody-Rb complex; iv) contacting the capture antibody-Rb complex with an anti-Rb primary antibody and isolating the capture antibody-Rb-primary antibody complex; and v) measuring the amount of CDK-phosphorylated Rb in the sample by quantitating the amount of said primary antibody in the capture antibody-Rb-primary antibody complex; wherein the agent modulates CDK activity if the amount of the CDK-phosphorylated Rb differs from a control cell lysate not contacted with the agent.

In a preferred embodiment, the method identifies a CDK inhibitor for the treatment of a disease or condition that is improved by inhibiting cellular proliferation, for example, cancers, autoimmune diseases, inflammatory disorders, degenerative disorders, such as macular degeneration and nephropathy, cardiovascular diseases, stroke, viral diseases, fungal diseases, and dermatological disorders, such as psoriasis. In an alternative preferred embodiment, the method identifies a CDK agonist for the treatment of a disease or condition that is improved by increasing cellular proliferation, for example, neurodegenerative diseases, such as Alzheimer's disease, or to stimulate wound healing or immune system activity.

In other preferred embodiments of the second aspect of the invention, the capture antibody is bound to a test plate, preferably a multi-well test plate, and the CDK activity measured is human CDK. Preferably, the method assays CDK2 activity or CDK4 activity. In other preferred embodiments, the agent is contacted with cultured cells or the agent is administered to an animal and the *ex vivo* activity is measured in a tissue sample taken from the animal. An additional preferred embodiment of the second aspect of the invention is its incorporation into a high throughput screen. This screen involves testing a plurality of agents, with at least one agent in each of a plurality of cell samples. For convenience, agents may also be initially pooled together in a single cell sample to test their effects.

By "cyclin-dependent kinase activity" or "CDK activity" is meant the enzymatic activity that results in the phosphorylation of retinoblastoma (Rb) protein at one or more of the following serine (Ser) or threonine (Thr) residues: Ser249; Thr252; Thr356; Ser612; Ser780; Ser807; Ser811; and/or Thr 821. Examples of CDKs that have such activity include CDK2, which phosphorylates Ser249, Thr252, Thr356, Ser612, Ser807, Ser811, and Thr 821, and CDK4, which phosphorylates Ser780.

By "capture antibody" or "anti-phosphoRb antibody" is meant an antibody that specifically recognizes Rb protein, but only if the Rb is phosphorylated by CDK activity at one or more of the following serine (Ser) or threonine (Thr) residues: Ser249; Thr252; Thr356; Ser612; Ser780; Ser807; Ser811; and/or Thr 821.

By "anti-Rb primary antibody" is meant an antibody that specifically recognizes Rb protein, but in a phosphorylation-independent manner.

By "quantitating the amount of primary antibody" is meant a method of measuring the amount of anti-Rb primary antibody using a detectable label such as alkaline phosphatase, horseradish peroxidase, β-galactosidase, biotin, Europium or a radiolabel. The label may be conjugated to a secondary antibody that specifically recognizes the primary antibody. Alternatively, the label may be conjugated to the primary antibody. In the case of alkaline phosphatase, horseradish peroxidase, or β-galactosidase, the method of quantitation relies on the use of a substrate that produces a chemiluminescent, chromogenic, or fluorescent signal in response to the enzymatic activity. In the case of biotin, the quantitation relies on the interaction between biotin and either streptavidin or avidin. And, in the case of radiolabel, quantitation relies on radioactive counting or autoradiography.

By "differs" is meant varying in a statistically significant manner (p<0.1). The probability that the variation between samples is nonrandom can be determined by statistical calculations well known in the art.

By "increases" or "decreases" CDK activity is meant a statistically significant variation in mean value above or below, respectively, the control mean value. Preferably, the variation from the control mean value is a least 25%, more preferably, at least 50%, and most preferably, at least 75%.

The invention provides a number of advantages. For example, the present method can be used to provide information regarding the level of intracellular CDK activity. The method provides more physiologically relevant information on CDK activity than other methods known in the art, which typically analyze *in vitro* CDK activity. Therefore, the present method is useful when studying changes in intracellular CDK activity over the course of the cell cycle or to identify compounds that modulate CDK activity in cultured cells or in animals.

The identification of novel CDK inhibitors also provides the benefit of creating a pool of cell cycle regulators that can be used to modulate cellular proliferation for the following therapeutic advantages: 1) when used as antitumor agents, the CDK inhibitors generate little risk of secondary tumor development because they lack direct DNA interaction; 2) the CDK inhibitors provide candidate therapies to treat conditions (e.g., viral infections) in which CDK activity is not misregulated, but is nonetheless essential for maintenance of the condition; and 3) the CDK inhibitors may be used to protect normal cells from the toxicity of cycle-specific chemotherapeutic agents which occurs in S-phase, G2, or mitosis, by preventing cell cycle progression in the normal cells (Stone et al., Cancer Research 56: 3199-202, 1996; Kohn et al., Journal of Cellular Biochemistry 54: 440-52, 1994).

Other features and advantages of the invention will be apparent from the following detailed description and from the claims. While the invention is described in connection with specific embodiments, it will be understood that it is capable of further modifications. Therefore, this application is intended to cover any variations, uses, or adaptations of the invention that follow, in general, the principles of the invention, including departures from the present disclosure that come within known or customary practice within the art. All publications mentioned in this description are herein incorporated by reference.

### List of Figures and Sequences

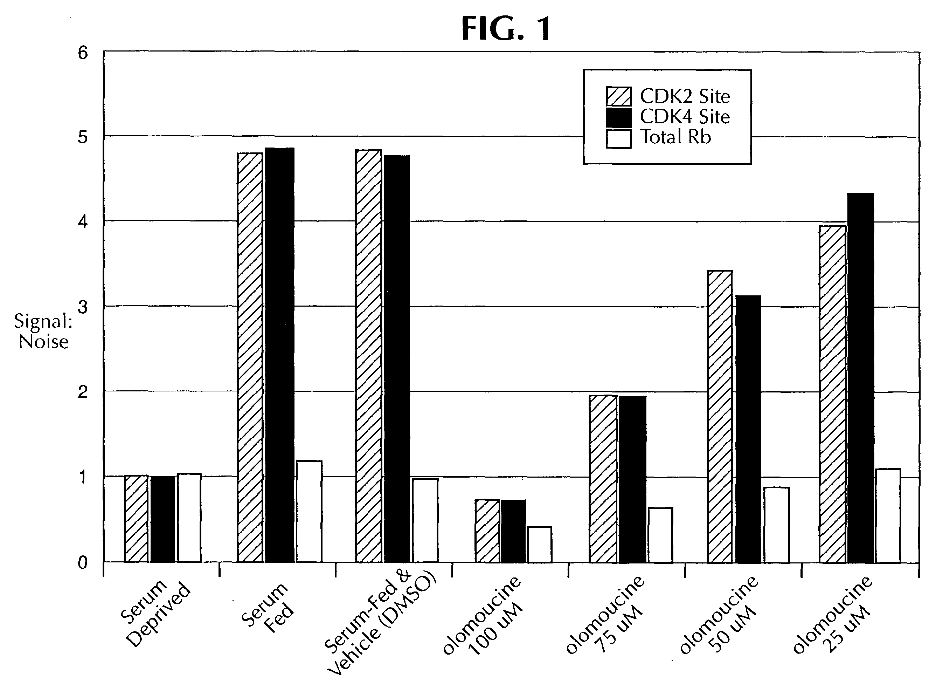

Figure 1 shows the results of a CDK activity assay in a breast carcinoma cell line.

SEQ ID NO: 1 shows the peptide sequence used in phosphorylated form to generate antibodies recognising Rb protein phosphorylated at position Ser249 and Thr252.

SEQ ID NO: 2 shows the peptide sequence used in phosphorylated form to generate antibodies recognising Rb protein phosphorylated at position Thr356.

SEQ ID NO: 3 shows the peptide sequence used in phosphorylated form to generate antibodies recognising Rb protein phosphorylated at position Ser612.

SEQ ID NO: 4 shows the peptide sequence used in phosphorylated form to generate antibodies recognising Rb protein phosphorylated at position Thr821.

### Description of the Figures

Figure 1 is a bar graph that demonstrates the results of an ELISA-based CDK activity assay in T47D cells. The assay reveals a significant increase in cellular CDK2 and CDK4 activity following serum readministration in serum-deprived cells (serum-fed) as compared to cells that remain serum-deprived (serum-deprived). However, the assay also reveals a dose-dependent inhibition of this serum-induced effect in cells treated with 25-100 µM olomoucine dissolved in a dimethylsulfoxide (DMSO) vehicle.

### Detailed Description

### Assay Protocol

The present invention features methods for measuring CDK activity by quantifying the amount of retinoblastoma (Rb) protein that is phosphorylated by CDK activity at one or more of the following serine (Ser) or threonine (Thr) residues: Ser249; Thr252; Thr356; Ser612; Ser780; Ser807; Ser811; or Thr 821. Examples of specific CDKs that can be assayed by the present methods include CDK2 (which phosphorylates Rb protein at Ser249, Thr252, Thr356, Ser612, Ser807, Ser811, and Thr 821) and CDK4 (which phosphorylates Rb protein at Ser780). Of particular note, the methods of the present invention can be used in a cell-based assay to assess intracellular CDK activity. Thus, the present methods may be used, for example, to assess CDK activity in cultured cells derived from an immortalized cell line, a primary cell line, or from a tissue sample excised from an animal, preferably, a mammal. In addition, the methods of the present invention can also be used in screening assays to identify agents that modulate CDK activity in cultured cells or in the cells of an animal by pre-exposing the cells or animal to the agent. Preferably, the screening assays are high-throughput assays, and the animals represent a disease model, such as a rodent tumor model.

As a first step in quantitating CDK-phosphorylated Rb in a sample, the methods of the present invention employ the use of "capture" antibodies that specifically react with Rb proteins that are phosphorylated at one or more of the following residues: Ser249; Thr252; Thr356; Ser612; Ser780; Ser 807; Ser811; and Thr821. For example, CDK2 activity is assessed using one of the following antibodies: an antibody specific for Rb phosphorylated at Ser807 and/or Ser811 (Cat. #9308, New England BioLabs, Inc., Beverly, MA); one specific for Rb phosphorylated at Ser249 and/or Thr252 (e.g., made with the following KLH-conjugated peptide antigen: Acetyl-NG[pS]PR[pT]PRRGQNC-amide, based on SEQ ID NO: 1), one specific for Rb phosphorylated at Thr356 (e.g., made with the following KLH-conjugated peptide antigen: Acetyl-FETQR[pT]PRKSNLDC-amide, based on SEQ ID NO: 2), one specific for Rb phosphorylated at Ser612 (e.g., made with the following KLH-conjugated peptide antigen: YLSPVR(pS)PKKKGST, based on SEQ ID NO: 3), or one specific for Rb phosphorylated at Thr821 (e.g., made with the following KLH-conjugated peptide antigen: SEGLP(pT)PTKMTPRS, based on SEQ ID NO: 4). Similarly, an antibody specific for Rb phosphorylated at Ser780 (e.g., Cat. #9307S, New England Biolabs, Beverly, MA; Code # 555, Medical and Biological Laboratories, Nagoya, Japan) is employed to assess CDK4 activity. It is preferred that the capture antibody be polyclonal.

After the CDK-phosphorylated Rb protein in the test sample is complexed with the capture antibody, the complex is isolated from the remaining sample. To isolate this complex, it is preferred that the capture antibody is first attached to a solid phase support, for example, a multi-well plate or a bead, by standard methods known in the art (see, e.g., Current Protocols in Immunology, John Wiley and Sons, New York, NY, 1999). Examples of solid phase supports that can be used for antibody attachment include, in order of preference, Nunc Maxisorb plates, Nunc Polysorb plates, and protein A coated plates (VWR Scientific, Bridgeport, NJ). To attach the capture antibody to the solid phase support, the capture antibody is diluted in a buffer such as 0.05 M carbonate-bicarbonate pH 9.6 (Cat. # C-3041, Sigma Chemical Co., St. Louis, MO) to approximately 10 µg/ml. This antibody dilution is then applied to the plate and incubated for a time ranging from approximately 4 hours to overnight at 4°C. The antibody-bound support is washed in a 25 mM Trizma base (Tris) or a 50 mM N-(2-hydroxyethyl)-N'-(2-ethanesulfonic acid) (Hepes) based buffer (pH 7.4-7.8) containing salt (e.g., 100-150 mM NaCI) and a small amount of detergent (e.g., 0.02-0.1 % Tween-20) (all of the wash buffer components are available from Sigma Chemical Co., St. Louis, MO). An exemplary wash buffer is an imidazol wash buffer (Cat. # 50-63-01, KPL, Inc., Gaithersburg, MD). (In the event that an alkaline phosphatase detection system is used in a subsequent step of this assay, phosphate buffered saline is not recommended as an incubation or wash buffer.)

Following the wash step, the capture antibody-bound support is incubated with a blocking buffer for approximately 0.5-2 hours at room temperature. Exemplary blocking buffers include, in order of preference, Superblock #37515 (Pierce Chemical, Rockford, IL), SuperBlock #37535 (Pierce Chemical, Rockford, IL), 0.2% Casein (Sigma Chemical Co., St. Louis, MO) in Tris buffered saline (TBS) (137 mM NaCl, 3 mM KCI, 25 mM Tris Base pH7.6; all components available from Sigma Chemical Co., St. Louis, MO) and 0.1% Tween-20, and 0.05% skim milk in TBS and 0.1% Tween-20 (all of the incubation buffer components are available from Sigma Chemical Co., St. Louis, MO). Subsequent to the aspiration of the blocking buffer from the capture antibody-bound support, the next step is contact with the test sample.

When the intracellular activity of a CDK is measured by the methods of the present invention, the test sample comprises a cell lysate derived from a cell line or animal tissue that was subjected to the desired experimental conditions. Any cells and cell lines with normal growth regulation express Rb protein and CDKs, and can, therefore, be used in the present assay. Many tumor cell lines, such as U2OS osteosarcoma cells, Colo 205 colorectal carcinoma cells, and DLD-1 colorectal cells, also express Rb and CDKs and may be used. However, care must be taken to synchronize the tumor cells, if synchronization is relevant to the CDK to be assayed (as in the case of CDK2). Examples of tumor cell lines that can be synchronized by serum deprivation include T47D breast carcinoma cells and MDA-MB-231 breast carcinoma cells. It is preferred that cells can be synchronized within 24 hours of serum deprivation. An exemplary medium that is applied to cells to induce synchronization is Dulbecco's Modified Eagles Medium (DMEM) that is phenol red free (Cat. # 21063-029, Life Technologies, Rockville, MD), with 0.1% bovine serum albumin (BSA) (Cat. #A3294, Sigma Chemical Co., St. Louis, MO), P/S/G (2 mM L-glutamine, 100 units/ml penicillin, 100 µg/ml streptomycin) (Cat. #61146, Sigma Chemical Co., St. Louis, MO). If cells do not require synchronization, they may be cultured in standard growth media, such as DMEM (Cat. # 11965-092, Life Technologies, Rockville, MD), 10% fetal bovine serum (FBS)(Cat. #16140-071, Life Technologies, Rockville, MD), and P/S/G. Tumor cell lines and normal cell lines are publicly available from sources such as the American Type Culture Collection (ATCC, Manassas, VA) and Clonetics (Walkersville, MD).

Primary cell preparations, such as fibroblasts derived from rat or mouse embryonic tissue may also be used to assess CDK activity by the present methods. These fibroblasts may be isolated using the following exemplary procedure. Under sterile conditions the embryonic spleen, liver, heart, lungs, and (if more that 16 days old) head are removed. The remaining tissue is transferred to a Petri dish with 10-15 ml of a 0.04% trypsin solution (Gibco BRL, Grand Island, NY) at approximately 37°C. The tissue is minced finely, the tissue/trypsin mixture is transferred to a Erlenmeyer flask, and 20-30 ml of fresh trypsin is added to the flask. Following an incubation in a water bath (37°C) for approximately 30 minutes, the trypsin is removed and the suspended cells are removed from the flask and added to 50 ml centrifuge tubes. An equal volume of DMEM-high glucose (Life Technologies, Rockville, MD) with 10% FBS (DMEM-H + FBS) is added to the tubes. To the remaining tissue precipitate in the flask, 20-30 ml of trypsin is added and the trypsination step is repeated. The suspended cells are then aspirated, transferred to centrifuge tubes, and resuspended with an equal volume of DMEM-H + FBS. All of the cell suspensions are then centrifuged at 300xg for 10-15 minutes. The supernatant is aspirated off and the cells are resuspended in fresh DMEM-H + FBS. Cells may then be cultured in plates. The media is changed after 24 hours and the cells are passaged 1:3 when confluent. To store cells, they may be frozen when approaching confluence.

To conduct assays to determine whether an agent affects the activity of a CDK in a cell, the agent is first dissolved in a vehicle such as water, dimethylsulfoxide (DMSO), or trifluoroacetic acid (Sigma Chemical Co., St. Louis, MO). For application to cultured cells, the agent in vehicle is first diluted in cell growth medium to achieve the desired concentration or range of concentrations, which typically fall within the range of 0-100 µM. Examples of the growth medium used include DMEM, McCoy's medium, or minimal essential medium (Life Technologies, Rockville, MD). The chosen medium is optimized for the particular cell line used in the assay, and may include specific growth factors. For some cells such as keratinocytes, cell-specific optimal growth media are commercially available (Cat. # 3001, 3004, 3107, and 3115, Clonetics, Walkersville, MD).

Given that most cells have a 16-36 hour cell cycle, it is preferred that cultured cells are incubated with the agent for 16-24 hours, more preferably, 16-21 hours, in order to maximize any possible effect on cell cycle progression, as measured by CDK phosphorylation of Rb protein. The cells are then lysed in a detergent containing buffer, such as a 50 mM Hepes buffer pH 7.6, containing 0.1% NP-40, 250 mM NaCI, 5 mM NaF, 1 mM dithiothreitol (DTT) (all above components of the lysis buffer are available from Sigma Chemical Co., St. Louis, MO) and a combination of protease inhibitors. Typically, the combination of protease inhibitors includes inhibitors that are effective against serine-, cysteine-, and metallo-proteases in bacterial, mammalian, yeast, and plant cell extracts. An exemplary combination is pefabloc SC (0.1-1.0 mg/ml), leupeptin (0.5 µg/ml), aprotinin (0.06-2.0 µg/ml), and pepstatin (0.7 µg/ml) (all protease inhibitors are available from Roche Molecular Biochemicals, Indianapolis, IN). Commercially available combinations of protease inhibitors are mixed together in concentrated tablet form (Cat. #1697498, (Boehringer Mannheim) Roche Molecular Biochemicals, Indianapolis, IN) for convenient addition to a stock lysis buffer to create the desired protease combination. Another exemplary lysis buffer is a 25 mM Bicine buffer pH 7.6 (Cat. #78501T, Pierce Chemical, Rockford, IL), which can be used following the addition of DTT (1 mM) and a combination of the above-described protease inhibitors.

When assessing the effect of an agent following administration to a whole animal, the nu/nu mouse is the preferable cancer model to use. The mice are inoculated subcutaneously in the lower abdomen with approximately 5 million tumor cells (e.g., Colo 205 or HCT 116 colorectal carcinoma cells in a total volume of 200 µl phosphate buffered saline (Life Technologies, Rockville, MD). When the tumors grow to approximately 100-200 mm³, the agent is administered through the desired route, e.g., intraperitoneally, subcutaneously, intravenously, or orally. When the *in vivo* kinetics of the agents are unknown, it is preferred that they be administered continuously to achieve steady state before testing. For example, the agents may be administered by osmotic mini-pumps (Alzet Model 2001, 7-day pump, Alza Corp, Newark, DE) containing the test agent. These pumps are implanted in anesthetized mice (e.g., 50 mg/kg nembutal, intraperitoneal). The test agents are first dissolved in an appropriate vehicle such as cyclodextrin, propylene glycol:DMSO (1:1), or 5% gelucine, etc. Tissues from the animals are later studied, for example, on day 5 post-implantation. Tumor cells and/or other tissues are removed and stored in liquid nitrogen. Plasma is saved for analysis of agent concentration. Further analysis of CDK activity is then determined by assessing Rb protein phosphorylation, as described herein. Cells are first pulverized in a liquid nitrogen-cooled pulverizer and then suspended the previously described lysis buffer. Samples are vortexed and then centrifuged at 8,000xg for approximately 10 minutes at 4°C. The protein concentration of the supernatant is determined by protein assay (Cat. #500-0006, Biorad, Hercules, CA). Samples are diluted to 100 µg/ml and 100 µl/well is added to the multiwell test plate. In all cases the level of phosphorylated Rb is correlated with the plasma level of the agent to determine if the agent affects CDK level.

Whether the cell lysate is derived from cultured cells or a tissue sample, the cell lysate is added to the capture antibody-bound plates and is incubated for approximately 1-3 hours at room temperature. It is preferred that all incubations are performed on a low speed shaker at 30-200 revolutions/minute. The cell lysate is aspirated and an anti-Rb primary antibody, diluted to approximately 1-5 µg/ml in an incubation buffer such as 1% casein in TBS pH 7.4-7.8, with or without 0.1% Tween-20, is added to the capture antibody-bound plates.

The primary antibody recognizes Rb protein in a phosphorylation-independent manner. Exemplary antibodies include a monoclonal antibody directed against a human Rb epitope between residues 300-380 (Cat. #14001A, Pharmingen, San Diego, CA) and a monoclonal antibody directed against a human Rb epitope between residues 612-928 (Cat. #MK-15-1, Medical & Biological Laboratories, Nagoya, Japan).

Depending on the antibody used, the antibody solution is incubated with the capture antibody-bound plate for approximately 1-3 hours at room temperature on a low speed shaker. In the case of the Pharmingen antibody (Cat. #14001A), the preferred incubation period is 2 hours. Following incubation, the plates are then washed multiple times (e.g., 3-6 times) with the previously described wash buffer.

Upon completion of this series of reagent incubations, the solid phase support contains bound capture antibody, complexed with CDK phosphorylated Rb protein, complexed with the primary antibody. To quantify the amount of CDK phosphorylated Rb protein that was originally present in the test sample, the amount of primary antibody contained in the plate-bound antibody complex is assessed using a standard detection system. This system of detection incorporates the use of a detectable label that is either conjugated to the primary antibody or to a secondary antibody that specifically recognizes the primary antibody. Examples of detectable labels include peroxidases, such as horseradish or soybean peroxidase, alkaline phosphatase, β-galactosidase, chelated lanthanides, biotin, Europium and radiolabels.

Given the commercial availability of secondary antibodies conjugated to detectable labels, the use of one of these antibodies is the preferred method for detecting the primary antibody. Examples of secondary antibodies that are available for use in the present invention include a donkey anti-mouse alkaline phosphatase-labelled antibody (Cat. #715-055-150, Jackson ImmunoResearch Laboratories, West Grove, PA), or a goat anti-mouse horseradish peroxidase-labelled antibody (Cat. #12-349, Upstate Biotechnologies, Lake Placid, NY, and Cat. # 10767, Sigma Chemical Co., St. Louis, MO)), or a rabbit anti-mouse Europium-labelled antibody.

Alternatively, the primary antibody could itself be conjugated with a detectable label. Methods for conjugating alkaline phosphatase, horseradish peroxidase, or β-galactosidase to an antibody, whether a primary or secondary antibody, are well-described in the literature (see, e.g., Porstmann et al., J. Immun. Meth. 79: 27-37, 1985; Imagawa et al., J. Appl. Biochem. 4: 41-57, 1982). In addition, the antibody of choice could be radiolabelled (Goding, Monoclonal Antibodies: Principles and Practice, Academic Press, Inc., San Diego, CA, 1996, 3^{rd} ed.), or biotinylated (Ignatowski et al., J. Exp. Ther. 290: 863-70, 1999; Southwick et al., Cytometry 11: 418, 1990). Kits for conjugating a peroxidase or alkaline phosphatase label to an antibody are commercially available from Pierce Chemical (e.g., Cat. #'s 31487, 31488, and 31489, Rockford, IL). Similarly, kits are commercially available to conjugate an antibody to a biotin label (e.g., Cat. #C5585, Sigma Chemical Co., St. Louis, MO) or to a Europium label (e.g., Cat. # 1244-302, Wallac).

Exemplary methods for detecting the presence of such labelled antibodies are described in the literature (see, e.g., Using Antibodies: A Lab Manual, eds. Harlow and Land, Cold Spring Harbor Laboratories, Cold Spring Harbor, NY, 1999, and Current Protocols in Immunology, Cell Biology, and Protein Science, John Wiley & Sons, New York, NY, 1999). Briefly, the detection of the horseradish peroxidase label is achieved using a chromogenic substrate, 2,2'-azino-bis(3-ethylbenzothiazoline-6-sulfonic acid) diammonium salt (Cat. # 11557, Fluka (Sigma Chemical Co., St. Louis, MO)), as further described in Szutowicz et al., Anal. Biochem. 138: 86, 1984, Envall, Methods Enzymol. 70: 419, 1980, and AI-Kaissi and Mostratos, J. Immunol. Methods 58: 127, 1983. To detect the alkaline phosphatase label, a chromogenic substrate, nitroblue tetrazolium chloride 5-bromo-4-chloro-3-indolyl phosphate toluidine salt (BCIP-NBT) (Sigma Chemical Co., St. Louis, MO) can be used, or the chemiluminescent substrate CDP-Star Sapphire II (Cat. #MS100RX, Tropix, Bedford, MA) can be used, as described, for example, in Thompson and Larson (BioTechniques 12: 656, 1992). Europium chelates require an enhancement solution (Cat. #1244-114, Wallac). Given the sensitivity of an ELISA-based assay using a luminescent detection system, as further described in the example below, it is the preferred method of detecting CDK activity.

### Test Agents or Compounds

A test agent or compound is identified as a CDK modulator if it changes the level of CDK-phosphorylated Rb in a sample in a statistically significant manner (p<0.1) as compared to a control sample, and produces a statistically significant change above or below the control mean value.

In general, agents or compounds which modulate CDK activity are identified from libraries of natural products, or synthetic (or semi-synthetic) extracts, chemical libraries, or individual compounds, and are produced according to methods known in the art. Examples of such extracts or compounds include, but are not limited to, naturally-occurring or synthetic chemical compounds, small organic or inorganic molecules, biological macromolecules (e.g., peptides, proteins, and nucleic acids), mixtures of chemical compounds, plant-, fungal-, prokaryotic-, or animal-based extracts, and fermentation broths.

Numerous methods are available for generating random or directed synthesis (semi-synthesis or total synthesis) of any number of chemical compounds, including, but not limited to, saccharide-, lipid-, peptide-, and nucleic acid-based compounds. The assays described herein are particularly useful in performing high-throughput screening assays, which accommodate the screening of large compound libraries.

Synthetic compound libraries are also commercially available, for example, from Brandon Associates (Merrimack, NH) and Aldrich Chemical (Milwaukee, WI). Libraries of natural compounds in the form of bacterial, fungal, plant, and animal extracts are commercially available from a number of sources, including Biotics (Sussex, UK), Xenova (Slough, UK), Harbor Branch Oceanographics Institute (Ft. Pierce, FL), and PharmaMar, U.S.A. (Cambridge, MA). In addition, natural and synthetic libraries are produced, if desired, according to methods known in the art, for example, by standard extraction and fractionation methods, organic synthesis, and methods of combinatorial chemistry.

When a crude extract is found to modulate CDK activity, further fractionation can be used to isolate and purify the chemical constituents responsible. Methods of fractionation and purification of such heterogeneous extracts will be known in the art in light of this disclosure. If desired, compounds identified as CDK modulators can be further modified, according to methods known in the art in light of this disclosure, to enhance their modulating effect or to reduce toxicity.

### Therapeutic Applications for Modulators of CDK Activity

Agents or compounds identified by the methods of the present invention as agonists or antagonists of CDK activity have several therapeutic applications. For example, agents or compounds identified as CDK inhibitors are useful in the treatment of diseases such as cancers, autoimmune diseases, viral diseases, fungal diseases, degenerative disorders, cardiovascular diseases, stroke, inflammatory disorders, and dermatological disorders, as well as other diseases and conditions in which the inhibition of cellular proliferation is beneficial.

The CDK inhibitors are useful in the treatment of a variety of cancers, including the following: carcinoma, including that of the bladder, breast, brain, colon, kidney, liver, lung (including small cell lung cancer), esophagus, gall bladder, ovary, pancreas, stomach, cervix, thyroid, prostate, and skin (including squamous cell carcinoma), hematopoietic tumors of lymphoid lineage (including leukemia, acute lymphocytic leukemia, acute lymphoblastic leukemia, B-cell lymphoma, T-cell lymphoma, Hodgkins lymphoma, non-Hodgkins lymphoma, hairy cell lymphoma and Burkett's lymphoma), hematopoietic tumors of myeloid lineage (including acute and chronic myelogenous leukemias, myelodysplastic syndrome and promyelocytic leukemia), tumors of mesenchymal origin (including fibrosarcoma and rhabdomyosarcoma), tumors of the central and peripheral nervous system (including astrocytoma, neuroblastoma, glioma and schwannomas), and other tumors (including melanoma, seminoma, teratocarcinoma, osteosarcoma, xenoderoma pigmentosum, keratoacanthoma, and thyroid follicular cancer).

In addition to inhibiting the proliferation of cancerous cells, CDK inhibitors could act as reversible cytostatic agents which may be useful in the treatment of any disease or condition associated with abnormal or deleterious cellular proliferation, such as Kaposi's sarcoma, benign prostate hyperplasia, familial adenomatosis polyposis, neurofibromatosis, atherosclerosis, thrombotic microangiopathy syndromes, pulmonary fibrosis, arthritis, psoriasis, restenosis following angioplasty or vascular surgery, hypertrophic scar formation, inflammatory bowel disease, transplantation rejection, endotoxic shock, mesangial cell proliferative disorders (including glomerulonephritis), diabetic nephropathy, glomerulopathies, malignant nephrosclerosis, organ transplant rejection, macular degeneration, fungal infections, and viral infections.

Of course, compounds identified as CDK agonists could also be used as therapeutic agents for diseases or conditions in which the stimulation of cellular proliferation is desired, such as to treat neurodegenerative disorders, for example, Alzheimer's disease, and also to stimulate wound healing and to stimulate immune system activity.

Agents that modulate CDK activity may be administered by any appropriate route. For example, administration may be parenteral, intravenous, intra-arterial, subcutaneous, intramuscular, intracranial, intraorbital, ophthalmic, intraventricular, intracapsular, intraspinal, intracisternal, intraperitoneal, intranasal, aerosol, by suppositories, or oral administration.

When administering therapeutic formulations comprising CDK modulators, the formulations may be in the form of liquid solutions or suspensions; for oral administration, formulations may be in the form of tablets or capsules; and for intranasal formulations, in the form of powders, nasal drops, or aerosols.

Methods well known in the art for making formulations are found, for example, in Remington's Pharmaceutical Sciences (ed. Gennaro, Mack Publishing Co., Easton, PA, 18^{th} ed., 1990)

### Example: ELISA-based Assay for CDK2 or CDK4 Activity

A solution containing a capture antibody diluted to 10 µg/ml in a 0.05 M carbonate-bicarbonate buffer pH 9.6 (Cat. #C3041, Sigma Chemical Co., St. Louis, MO) was applied to coat the wells of 96-well plates (100 µl/well, Nunc F96 MaxiSorp, VWR Scientific, Bridgeport, NJ) and incubated overnight at 4°C. For CDK2 assays, anti-phosphoRb antibody, which specifically recognizes Rb phosphorylated at Ser807 and Ser811, was used (Cat. #9308L, New England Biolabs, Inc., Beverly, MA); for CDK4 assays, anti-phosphoRb antibody, which specifically recognizes Rb phosphorylated at Ser 780, was used (Cat. #9307S, New England Biolabs, Inc., Beverly, MA, or Code #555, Medical and Biological Laboratories, Nagoya, Japan).

Breast carcinoma cells, T47D (HTB-133, ATCC, Manassas, VA, Depositor: I. Keydar), were cultured in 100 mm plates, trypsinized prior to confluence, and seeded in 96-well plates at 9,000 cells/200 µl/well in DMEM (Cat. # 11965-092, Life Technologies, Rockville, MD), 10% fetal bovine serum (FBS) (Cat. #16140-071, Life Technologies, Rockville, MD), P/S/G (2 mM L-glutamine, 100 units/ml penicillin, 100 µg/ml streptomycin) (Cat. # G1146, Sigma Chemical Co., St. Louis, MO).

For CDK2 assays, cells were cultured for one day and then synchronized by serum starvation as follows. Cell medium was aspirated, the cells were washed five times in Hanks Buffered Saline Solution (Cat. # 14025-092, Life Technologies, Rockville, MD). Cells were then incubated in DMEM without phenol red (Cat. #21063-029, Life Technologies, Rockville, MD), 0.1% BSA (Cat. #A3294, Sigma Chemical Co., St. Louis, MO), and P/S/G for 24 hours. While control cells were maintained in 0.1% BSA/DMEM to establish a baseline (serum-deprived), test cells were refed with DMEM, 1% FBS, P/S/G, and incubated for 20-24 hours with 0, 25 µM, 50 µM, or 100 µM olomoucine (Promega, Madison, WI) a known CDK inhibitor.

For CDK4 assays, cell synchronization was not required. Cells were cultured for two days in DMEM (Cat. #11965-092, Life Technologies, Rockville, MD), 10% FBS, P/S/G. The cell medium was then aspirated and 200 µl fresh medium with 0, 25 µM, 50 µM, or 100 µM olomoucine was added for a 20-24 hour incubation.

For both the CDK2 and CDK4 assays, olomoucine was first dissolved in a DMSO vehicle (Sigma Chemical Co., St. Louis, MO) at concentrations ranging of 0, 25 mM, 50 mM, and 100 mM in the solvent. These solutions were then diluted 1:1000 in DMEM, 10% FBS, P/S/G before the olomoucine or control solution was added to the cells.

The cells plated in 96-well plates were washed three times in cold TBS (as previously described) and lysed with 120 µl/well cold lysis buffer consisting of 50 mM Hepes buffer pH 7.6, 0.1% NP-40, 250 mM NaCI, 5 mM NaF, 1 mM DTT, and the protease inhibitors provided in protease tablets (Cat. #1697498, (Boehringer Mannheim) Roche Molecular Biochemicals, Indianapolis, IN). The cell lysates were maintained at 4°C for the duration of the following blocking step performed on the capture antibody-bound plates. The plates were washed two times with 250 µl/well imidazol wash buffer (Cat. # 50-63-01, KPL, Inc., Gaithersburg, MD), and incubated with blocking buffer (Cat. # 37515, Pierce Chemical, Rockford, IL) for one hour at room temperature.

Upon completion of the blocking step, the blocking buffer was aspirated and 100 µl of cell lysate was transferred to each well of the capture antibody-coated plates. The plates were placed on a shaker at the low speed setting (approximately 100 revolutions/minute) for a two hour incubation at room temperature. The plates were then washed six times in the above-described imidazol wash buffer.

The plates, with bound capture antibody-Rb complexes, were incubated with an anti-Rb monoclonal antibody (phosphorylation-independent) that recognizes an Rb epitope between amino acids 300-380 (Cat. # 14001A, PharMingen, San Diego, CA). This primary antibody was diluted to 2 µg/ml in 1% casein, TBS pH 7.6, 0.1% Tween-20 (all incubation buffer components are available from Sigma Chemical Co., St. Louis, MO). One hundred microliters of the solution was added to each well, and the plates were then incubated on the shaker at the low speed setting for two hours at room temperature. The plates were then washed six times with the imidazol wash buffer to isolate the capture antibody-Rb-primary antibody complex bound to the plate.

The final antibody incubated on the plate was a donkey anti-mouse alkaline phosphatase-labelled secondary antibody (Cat. #715-055-150, Jackson ImmunoResearch Laboratories, Inc., West Grove, PA). The secondary antibody was diluted 1:2000 in the incubation buffer consisting of 1% casein, in TBS pH 7.6, 0.1% Tween-20, and 100 µl of the solution was added to each well. Following the incubation of the plates on the shaker at the low speed setting for one hour at room temperature, the plates were again washed six times with the imidazol wash buffer to isolate the capture antibody-Rb-primary antibody-secondary antibody complex.

In the final step of the assay, the amount of CDK-phosphorylated Rb protein present in the original cell lysate sample was measured by quantifying the amount of labelled secondary antibody present on the plate. To each well, 100 µl of the alkaline phosphatase substrate CDP-Star Sapphire II (Cat. #MS100RX, Tropix, Bedford, MA) was added, the plates were incubated on a shaker for 20 minutes at room temperature, and the luminescent signal was then measured on a luminometer (Dynex Technologies, formerly Dynatech Laboratories, Chantilly, VA).

As shown in Fig. 1, intracellular CDK2 and CDK4 activity increased following the administration of nutrients in serum-starved cells, and also decreased in a dose-dependent manner following contact with a known inhibitor of CDK activity, olomoucine. These results demonstrate the usefulness of the present assay methods in measuring physiologically relevant changes in CDK activity associated with cell cycle regulation. The reduction in CDK activity following cell contact with olomoucine demonstrates that the assay can successfully detect agent-induced changes in CDK activity at the cellular level. The present assay methods are useful in screening assays to identify modulators of CDK activity, especially those that can modulate CDK activity at the intracellular level.

## Claims

1. A method for measuring cyclin-dependent kinase (CDK) activity in a sample, said method comprising:
i) contacting said sample with an anti-retinoblastoma protein (Rb) capture antibody and isolating the capture antibody-Rb complex;
ii) contacting said capture antibody-Rb complex with an anti-Rb primary antibody and isolating the capture antibody-Rb-primary antibody complex; and
iii) measuring the amount of CDK-phosphorylated Rb in said sample by quantitating the primary antibody present in said capture antibody-Rb-primary antibody complex.

2. The method of claim 1, wherein said CDK is CDK2.

3. The method of claim 1, wherein said CDK is CDK4.

4. The method of claim 1, wherein said method measures intracellular CDK activity.

5. The method of claim 4, wherein said method measures CDK activity in a cultured cell.

6. The method of claim 4, wherein said method measures *ex vivo* CDK activity in a cell taken from an animal.

7. The method of claim 1, wherein said CDK activity is human CDK activity.

8. The method of claim 1, wherein said capture antibody is bound to a test plate.

9. A method of identifying an agent that modulates CDK activity in a cell, said method comprising:
i) contacting an agent with said cell;
ii) lysing said cell;
iii) contacting said cell lysate with an anti-retinoblastoma protein (Rb) capture antibody and isolating the capture antibody-Rb complex;
iv) contacting said capture antibody-Rb complex with an anti-Rb primary antibody and isolating the capture antibody-Rb-primary antibody complex; and
v) measuring the amount of CDK-phosphorylated Rb by quantitating the amount of primary antibody present in said capture antibody-Rb-primary antibody complex;
wherein said agent modulates CDK activity if the amount of said CDK-phosphorylated Rb differs from a control cell not exposed to said agent.

10. The method of claim 9, wherein said method identifies an agent that decreases CDK activity.

11. The method of claim 10, wherein said agent is identified for the treatment of a disease or condition that is improved by inhibiting cellular proliferation.

12. The method of claim 11, wherein said disease or condition is selected from the group consisting of cancers, autoimmune diseases, viral diseases, fungal diseases, degenerative disorders, cardiovascular diseases, stroke, inflammatory disorders, and dermatological disorders.

13. The method of claim 9, wherein said agent increases CDK activity.

14. The method of claim 13, wherein said agent is identified for the treatment of a disease or condition that is improved by increasing cellular proliferation.

15. The method of claim 14, wherein said agent is used to treat neurodegeneration, to stimulate wound healing, or to stimulate immune system activity

16. The method of claim 9, wherein said CDK is CDK2.

17. The method of claim 9, wherein said CDK is CDK4.

18. The method of claim 9, wherein said method measures intracellular CDK activity.

19. The method of claim 9, wherein said method measures CDK activity in a cultured cell.

20. The method of claim 4, wherein said method measures *ex vivo* CDK activity in a cell taken from an animal.

21. The method of claim 9, wherein said CDK activity is human CDK activity.

22. The method of claim 9, wherein said capture antibody is bound to a test plate.

23. The method of claim 9, wherein a plurality of agents are tested, each agent in at least one of a plurality of cell samples.
